# EUROPÄISCHE PATENTSCHRIFT

(11) **EP 1 719 450 B1**
(45) Veröffentlichungstag und Bekanntmachung des Hinweises auf die Patenterteilung: **15.10.2008**
(21) Anmeldenummer: 06008930.7
(22) Anmeldetag: 28.04.2006
(51) Int. Cl.: A61B 17/04

(54) **Vorrichtung zum Einbringen eines Ankerelements samt Faden in einen Knochen**
Device for introducing an anchor with thread into a bone
Dispositif pour introduire un élément d'ancrage avec un fil dans un os

(30) Priorität: 04.05.2005 DE 102005021885
(43) Veröffentlichungstag der Anmeldung: 08.11.2006
(73) Patentinhaber: Karl Storz GmbH & Co. KG, 78532 Tuttlingen (DE)
(72) Erfinder: Berberich, Sascha, 78532 Tuttlingen (DE); Baltes, Ina, 78532 Tuttlingen (DE)
(74) Vertreter: Weller, Wolfgang

(56) Entgegenhaltungen:
- WO-A-20/04062507
- US-A1- 2003 144 696

## Beschreibung

Die Erfindung betrifft eine Vorrichtung zum Einbringen eines Ankerelements samt Faden in einen Knochen zum Fixieren einer Sehne oder eines Bandes, ohne Bewerkstelligen einer Bohrung im Knochen, mit einem Ankerelement und einem Werkzeug zum Einbringen des Ankerelementes samt Faden, wobei ein Abschnitt des Werkzeuges so ausgebildet ist, dass er von proximal nach distal in das Ankerelement einführbar ist, und wobei das Ankerelement ein Außengewinde und einen durchgehenden Kanal aufweist, in den das Werkzeug soweit einführbar ist, das ein distaler Endabschnitt des Werkzeuges in distaler Richtung über das Ankerelement hinausragt, und wobei der hinausragende distale Endabschnitt als stabiler Eintreiber ausgebildet ist und an seinem distalen Endabschnitt eine Spitze aufweist, und wobei das Ankerelement eine durchgehende Öffnung zur Aufnahme des Fadens aufweist, die quer zur Längsachse des Ankerelements verläuft.

Eine Vorrichtung zum Einbringen eines Ankerelements samt Faden mit diesen konstruktiven Merkmalen ist aus der WO 2004/062507A2 bekannt.

Derartige Ankerelemente werden samt einem Faden in einem Knochen verankert und mit dem Faden wird eine abgerissene Sehne oder ein Band wieder an dem Knochen fixiert.

Ein Haupteinsatzgebiet für solche Ankerelemente ist das Fixieren von abgerissenen Sehnen im Schulterbereich.

Es haben sich in dieser Technologie zwei Vorgehensweisen etabliert.

In der US 5,690,676 ist eine Ausführung beschrieben, bei der das Ankerelement einen durchgehenden Kanal aufweist, durch den ein Führungsdraht durch das Ankerelement hindurchgeschoben werden kann, so dass ein distaler Endabschnitt des Führungsdrahtes hinausragt. Dieser hinausragende Abschnitt dient insbesondere bei kleinen Ankerelementen und sehr kleinen Bohrungen als Ziel- oder Einführhilfe. Das bedeutet, der Draht wird in die Bohrung im Knochen eingesetzt und dann das Ankerelement über den Draht vorgeschoben und in die Bohrung eingeführt. Dann wird der Draht abgezogen und das eigentliche Werkzeug aufgesetzt und der zuvor beschriebene Eintreibvorgang bewerkstelligt.

Bei dieser Technologie ist es zwingend notwendig, vorher eine Bohrung in den Knochen einzubringen.

Aus der US 4,632,100 ist eine Vorrichtung zum Einbringen eines Ankerelementes beschrieben, das ohne vorheriges Bewerkstelligen einer Bohrung eingesetzt werden kann.

Dazu ist das Ankerelement selbst an seiner distalen Spitze als Eintreiber ausgebildet. Dazu muss das Ankerelement sehr massiv und insbesondere aus Metall hergestellt werden. Ferner ist an der Außenseite des Ankerelements ein Außengewinde vorgesehen, um das Ankerelement, nachdem es zunächst eingeschlagen wurde, durch eine Drehbewegung über das Außengewinde im Knochen zu verankern. Zu diesem Vorgang wird auf das proximale Ende des Ankerelements ein Werkzeug aufgesetzt. Auch hierbei kann gleichzeitig ein Faden mit eingebracht werden.

Nachteilig an dieser Ausführung ist, dass das Ankerelement sehr massiv, sehr aufwendig, insbesondere aus Metall gefertigt werden muss und als metallischer Fremdkörper im Körper verbleibt.

Derartige Schlaganker können nicht für osteoporotische Knochen eingesetzt werden. Ferner ist es ein Bestreben, resorbierbare Materialien einzusetzen, die nach und nach durch körpereigenes Knochenmaterial ersetzt werden können. Da resorbierbare Materialien aber nicht über eine ausreichende mechanische Stabilität verfügen, können Schlaganker aus solchen Materialien nicht eingesetzt werden.

Aus der US 2003/0144696 A1 ist ein Treiber bekannt, mit dem ein Anker ohne Bewerkstelligen einer Bohrung im Knochen in einen Knochen eingebracht werden kann. Der Anker weist einen Körper auf, der mit einem Gewinde versehen ist und einen durch den Körper hindurchgehenden Kanal aufweist. Der Treiber weist einen Abschnitt auf, der in den Kanal des Ankers von proximal nach distal so weit einführbar ist, dass ein distaler Endabschnitt des Treibers, der als eine Spitze ausgebildet ist, in distaler Richtung über den Anker hinausragt. Proximalseitig sind im Körper des Ankers Ausnehmungen und/oder Öffnungen vorgesehen, die zur Aufnahme eines Fadens dienen, wobei der Fadern derart in den Ausnehmungen und/oder Öffnungen aufgenommen ist, dass er durch den Kanal nicht verläuft.

In der eingangs genannten WO 2004/062507 A2 ist eine Vorrichtung dargestellt, mit der der Anker ohne Bewerkstelligen einer Bohrung im Knochen in einen Knochen eingebracht werden kann. Der Anker weist einen durch den Anker hindurchgehenden Kanal auf, in dem ein Abschnitt des Werkzeuges von proximal nach distal so weit einführbar ist, dass ein distaler Endabschnitt in distaler Richtung über den Anker hinausragt. Der hinausragende, als eine Spitze ausgebildete Endabschnitt ist als stabiler Eintreiber ausgebildet. Ferner weist der Anker eine Querbohrung zur Aufnahme eines Fadens auf. Beim Einbringen des Ankers in den Knochen wird der Faden bei einem Ausführungsbeispiel gleichzeitig durch die Querbohrung in dem Anker und eine Querbohrung im Werkzeug hindurchgeführt. Bei einem anderen Ausführungsbeispiel wird der Faden durch die Querbohrung in dem Anker durchgeführt und eine Schlaufe des durch den Anker durchgeführten Fadens wird in einem seitlichen Schlitz in der Vorrichtung aufgenommen.

Der vorliegenden Erfindung liegt die Aufgabe zugrunde, eine Vorrichtung der eingangs genannten Art dahingehend weiterzubilden, dass ein Verhaken oder Verklemmen der Spitze des Werkzeugs mit dem durch das Ankerelement quer durchgeführten Faden beim dessen Einbringen in den Knochen verhindert wird.

Erfindungsgemäß wird die Aufgabe dadurch gelöst, dass die Lage des Endpunktes der Spitze bezogen auf die Mittellängsachse des Werkzeugs radial versetzt ist.

Durch den seitlichen Versatz der Spitze wird verhindert, dass die Spitze auf den Faden trifft, der einmal quer durch das Ankerelement hindurchgeführt ist. Sie schiebt sich durch den seitlichen Versatz an dem Faden vorbei und verhindert ein Verhaken oder Verklemmen mit dem Faden.

Die durchgehende Öffnung zur Aufnahme des Fadens hat den Vorteil, dass sich der Faden einmal quer durch das Ankerelement erstreckt, so dass dann das entsprechende Widerlager vorhanden ist, über das der Faden anschließend mit der Sehne fest verbunden werden kann.

Das Versehen eines distalen Endabschnittes des Werkzeugs mit einer Spitze hat den Vorteil, dass über die Spitze vom Operateur exakt der Ansatz bzw. Einschlagpunkt angezielt werden kann und ferner ist ein sanftes Eintreiben des als stabiler Eintreiber ausgebildeten distalen Endabschnittes des Werkzeuges möglich.

Ferner ermöglicht die Ausbildung des Abschnittes des Werkzeugs, der über den Anker distal hinaussteht, als stabiler Eintreiber diesen Zusammenbau ohne vorheriges Einbringen einer Bohrung zunächst wie einen Schlaganker einzutreiben. Dabei wird aber nicht die distale Spitze des Ankerelementes eingetrieben, sondern nur der über das Ankerelement vorstehende distale Abschnitt des Werkzeuges. Dieser ist entsprechend fest, steif und auch aus metallischen Materialien auszubilden.

Durch das Vorsehen des Außengewindes an der Außenseite des Ankerelementes kann nach dem Einschlagen der distalen Spitze des Werkzeuges der Zusammenbau aus Ankerelement und Werkzeug gedreht werden und das Ankerelement in die Einschlagöffnung eingedreht und über das Außengewinde fixiert werden. Nach diesem Vorgang wird das Werkzeug abgezogen, und das Ankerelement sitzt fest verankert im Knochen, ohne dass dabei eine vorherige Bohrung notwendig war und ohne dass das Ankerelement aus stabilen insbesondere aus metallischen Materialien hergestellt werden musste.

In einer weiteren Ausgestaltung der Erfindung ist das Werkzeug drehschlüssig im Ankerelement aufgenommen.

Diese Maßnahme hat den Vorteil, dass die Drehbewegung beim Eindrehen des Gewindes des Ankerelements durch diesen Drehschluss einfach durchgeführt werden kann.

In einer weiteren Ausgestaltung der Erfindung ist das Ankerelement distal mit einer Verjüngung versehen.

Diese Maßnahme hat den Vorteil, dass das Ankerelement sanft gleitend über diese Verjüngung beim zunächst anfänglichen Eintreibvorgang in den Knochen eingetrieben werden kann.

In einer weiteren Ausgestaltung der Erfindung ist die Verjüngung so ausgebildet, dass diese durch die Spitze des Werkzeuges fortgesetzt wird.

Diese Maßnahme hat den besonderen Vorteil, dass ein sanfter Übergang von dem distalen Ende des Ankerelements zu der über dieses vorragende Spitze des Werkzeuges vorhanden ist, so dass der Eintreibvorgang besonders schonend durchgeführt werden kann.

In einer Ausgestaltung der Erfindung erstreckt sich das Außengewinde des Ankerelements über etwa die hintere proximale Hälfte des Ankerelementes.

Diese Maßnahme hat den Vorteil, dass das Ankerelement zunächst in dem linearen Eintreibvorgang bis zu einem gewissen Maß in den Knochen eingetrieben bzw. eingeschlagen werden kann, und erst anschließend sich das Außengewinde in den Knochen eindreht.

In einer weiteren Ausgestaltung der Erfindung erstreckt sich das Außengewinde des Ankerelements über die ganze Länge des Ankerelements.

Diese Maßnahme hat den Vorteil, dass bei besonders porösen Knochen, wenn eine Verankerung über die gesamte Außenseite des Ankerelementes notwendig ist, dies über dieses Außengewinde, das sich über den ganzen Körper erstreckt bewerkstelligt werden kann.

In einer weiteren Ausgestaltung der Erfindung weist der Abschnitt des Werkzeuges, der in das Ankerelement einsteckbar ist, ein Querschnittsprofil auf, das in etwa dreisternförmig ist.

Diese Maßnahme hat den Vorteil, dass über diese Geometrie eine sehr großflächige drehschlüssige Verbindung mit dem Ankerelement bewerkstelligt werden kann, so dass auch hohe Drehmomente nicht zu einer Verformung oder Abscheren des Ankerelements führen.

In einer weiteren Ausgestaltung der Erfindung weist der Abschnitt des Werkzeuges, der im Ankerelement eingeschoben ist, ein Querschnittsprofil auf, das in etwa achtförmig ist.

Diese Maßnahme hat den Vorteil, dass auch hier eine relativ großflächige drehschlüssige Anlage geschaffen werden kann, und dass in der Mitte der Acht ein Raum zur Verfügung steht, in dem der Faden beidseits Platz finden kann.

In einer weiteren Ausgestaltung der Erfindung geht die durchgehende Öffnung in Nuten über, die sich in axialer Richtung an der Außenseite des Ankerelements nach proximal erstrecken und zur Aufnahme des Fadens dienen.

Diese ebenfalls an sich bekannte Maßnahme hat den Vorteil, dass der Faden längs der Außenseite nach proximal geführt werden kann. Die Nuten erstrecken sich durch das Gewinde hindurch, so dass der Faden beim Eintreiben des Ankerelements über den Gewindeabschnitt nicht beeinträchtigt wird.

In einer weiteren Ausgestaltung der Erfindung sind am Werkzeug Nuten vorgesehen, die die Nuten des Ankerelements nach proximal fortsetzen und die der axialen Aufnahme des Fadens dienen.

Diese Maßnahme hat den Vorteil, dass der Faden durch die Ausrichtung der Nuten längs der Außenseite des Zusammenbaus von Ankerelement und Werkzeug nach proximal an beiden Seiten geführt werden kann.

In einer weiteren Ausgestaltung der Erfindung weist das Werkzeug in dem Abschnitt, in dem es in das Ankerelement eingeschoben ist, einen Fangschlitz zur Aufnahme des Fadens auf.

Diese Maßnahme hat den Vorteil, dass durch den Fangschlitz der in das Ankerelement eingeschobene Faden eingefangen und definiert im Werkzeug zum Liegen kommt.

In einer weiteren Ausgestaltung der Erfindung mündet der Fangschlitz zu einer Seite des Werkzeuges.

Diese Maßnahme hat den Vorteil, dass beim Einschieben des Werkzeugs in die durchgehende Öffnung im Ankerelement insbesondere durch die seitlich versetzte Spitze an eine Seite herangelegt wird und dann zwangsläufig in den Fangschlitz eingefädelt bzw. eingeschoben wird.

In einer weiteren Ausgestaltung der Erfindung kommt ein Ende des Fangschlitzes des Werkzeuges auf Höhe der durchgehenden Öffnung des Ankerelements zum Liegen, wenn das Werkzeug in das Ankerelement eingeführt worden ist.

Diese Maßnahme hat zum einen den Vorteil, dass der Faden in eine exakt vorbestimmte Position im Ankerelement vom Werkzeug gebracht wird, nämlich genau in Ausrichtung mit den seitlichen Öffnungen, durch die der Faden durch das Ankerelement hindurchtritt.

Gleichzeitig eröffnet diese Konstruktion auch die Möglichkeit, zunächst das Werkzeug in das Ankerelement einzuschieben und dann, durch das Fluchten von Öffnung und Ende des Fangschlitzes, den Faden durch den Zusammenbau durchzufädeln.

In einer weiteren Ausgestaltung der Erfindung weist das Werkzeug eine Schulter auf, die an einer entsprechenden Schulter am Ankerelement zum Liegen kommt.

Diese Maßnahme hat den Vorteil, dass die Einschubtiefe des Werkzeugs in das Ankerelement exakt begrenzt ist, nämlich, wenn die gegenseitigen Schultern aneinander liegen.

Es versteht sich, dass die vorstehend genannten und die nachstehend noch zu erläuternden Merkmale nicht nur in der angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine Seitenansicht eines erfindungsgemäßen Ankerelements,
- Fig. 2: eine perspektivische Seitenansicht des Ankerelements von Fig. 1 mit eingelegtem Faden,
- Fig. 3: einen Schnitt längs der Linie III-III in Fig. 1,
- Fig. 4: eine Seitenansicht des Ankerelements von Fig. 1 sowie eines distalen Bereiches eines Werkzeuges, das von proximal nach distal in das Ankerelement eingeschoben werden soll,
- Fig. 5: einen Zusammenbau des Ankerelements und des Werkzeugs von Fig. 4 samt eingelegtem Faden, und
- Fig. 6: eine Situation beim Einbringen des Ankerelements in einen Schulterknochen mit der erfindungsgemäßen Vorrichtung bestehend aus dem Zusammenbau aus Ankerelement, Werkzeug und Faden.

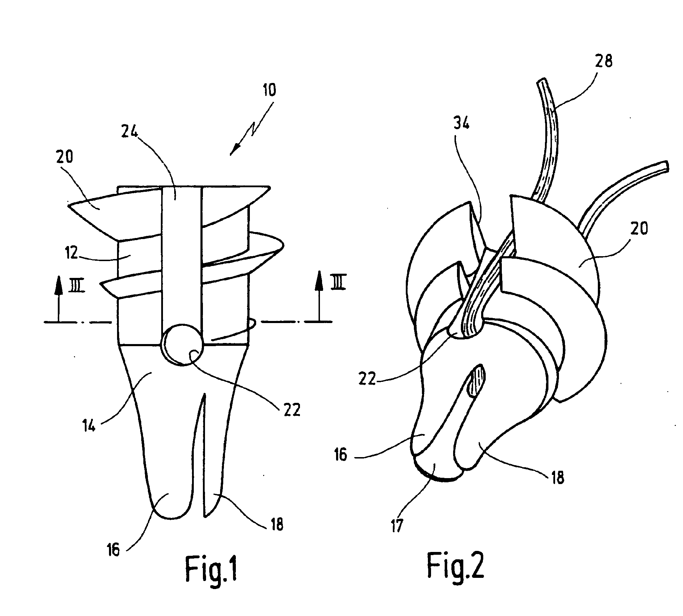

In den Fig. 1 bis 3 ist ein Ankerelement dargestellt, das in seiner Gesamtheit mit der Bezugsziffer 10 bezeichnet ist.

Das Ankerelement 10 weist einen etwa zylindrischen Körper 12 auf, der distalseitig in eine Verjüngung 14 übergeht.

Die Verjüngung 14 ist aus drei umfänglich gleichmäßig verteilten Segmenten 16, 17 und 18 zusammengesetzt.

An der Außenseite des zylindrischen Körpers 12 ist ein Gewinde 20 vorhanden.

Im Übergang von dem zylindrischen Körper 12 zur Verjüngung 14 ist eine quer durchgehende Öffnung 22 vorhanden.

Nach proximal erstrecken sich von der Öffnung 22 diametral gegenüberliegend Nuten 24 und 25, die proximal münden und sich durch das Gewinde 20 hindurch erstrecken.

Wie aus Fig. 2 zu entnehmen, kann ein Faden 28 durch die Öffnung 22 hindurchgeschoben werden und in die Nuten 24 und 25 eingelegt werden, so dass sich die beiden freien Fadenenden nach proximal vom Ankerelement 10 weg erstrecken. Der Faden erstreckt sich dabei diametral quer durch das Ankerelement 10 hindurch.

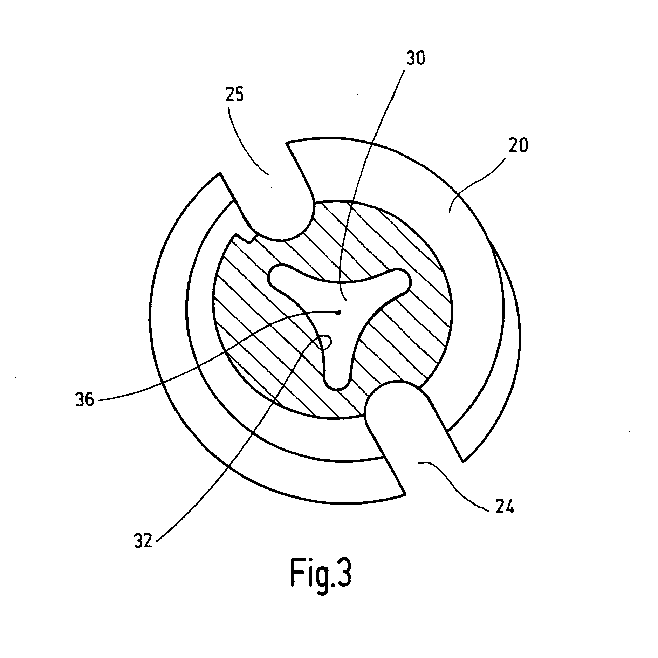

Aus der Schnittdarstellung von Fig. 3 ist zu erkennen, dass sich mittig durch das Ankerelement 10 ein durchgehender Kanal 30 erstreckt, der im Bereich des zylindrischen Körpers 12 einen Querschnitt 32 in Form eines Dreisternes aufweist.

Proximalseitig endet der Kanal 30 in einer abgeschrägten Schulter 34.

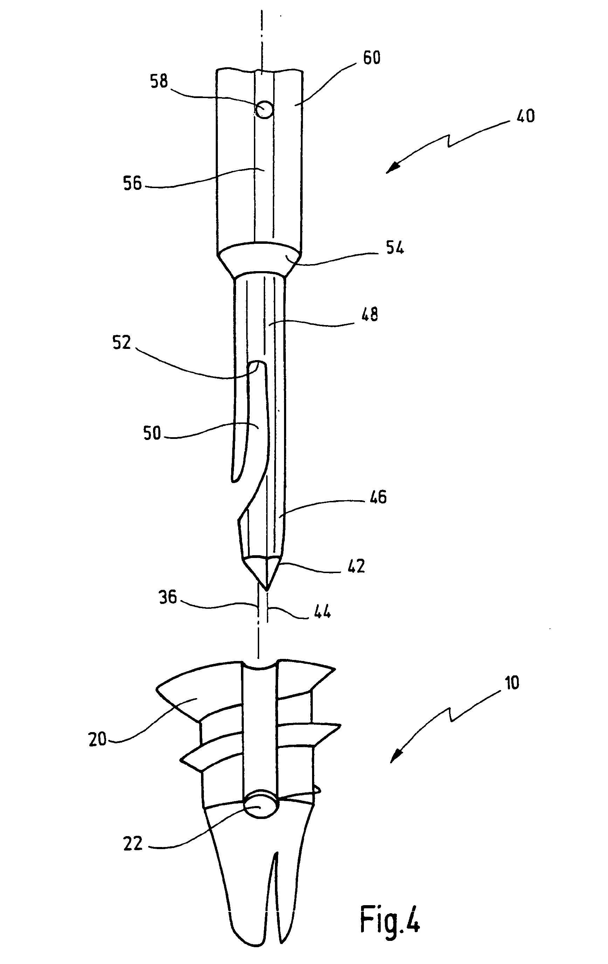

Wie aus Fig. 4 zu entnehmen, wird zum Einbringen des Ankerelements 10 von proximal nach distal ein Werkzeug 40 eingeschoben.

Das Werkzeug 40 weist distalseitig eine etwa dreikantige Spitze 42 auf, die relativ massiv ist. Das äußere Ende der Spitze 44 ist seitlich etwas gegenüber der Mittellängsachse 44 des Werkzeuges 40 und somit auch gegenüber der Mittellängsachse 36 des Ankerelements 10 versetzt. Die Spitze 42 geht in einen Abschnitt 48 über, dessen Querschnittsprofil ebenfalls dreisternförmig ist.

In anderen Worten ausgedrückt, kann der Abschnitt 48 formschlüssig und drehschlüssig in dem Querschnitt 32 des Kanals 30 des Ankerelements eingeschoben werden.

Wie aus Fig. 4 zu erkennen, ist in diesem Abschnitt 48 ein Fangschlitz 50 vorhanden, der zu einer Seite hin mündet und zwar zu der Seite, die gegenüberliegend zu der Versatzrichtung der Spitze 42 ist. Ein Ende 52 des Fangschlitzes 50 kommt, wie das nachfolgend noch beschrieben wird, wenn das Werkzeug 40 in das Ankerelement 10 eingeschoben ist, auf Höhe der durchgehenden Öffnung 22 des Ankerelements 10 zum Liegen.

Der Abschnitt 48 geht über eine Schulter 54 in einen stabförmigen Abschnitt 60 des Werkzeuges 40 über.

Die Schulter 54 ist so ausgebildet, dass sie passend an der Schulter 34 am proximalen Ende des Ankerelements 10 zum Liegen kommen kann. Dadurch wird die Einschubtiefe entsprechend begrenzt, bzw. bestimmt.

Aus Fig. 4 ist ferner zu erkennen, dass an der Außenseite des stabförmigen Abschnittes 60 Nuten 56, eingeschnitten sind, die die Nuten 24 und 25 am Ankerelement 10 verlängern. Von jeder Nut 56 steht radial ein Zapfen 58 vor, der zum Auffädeln oder Fixieren des Fadens 28 dient.

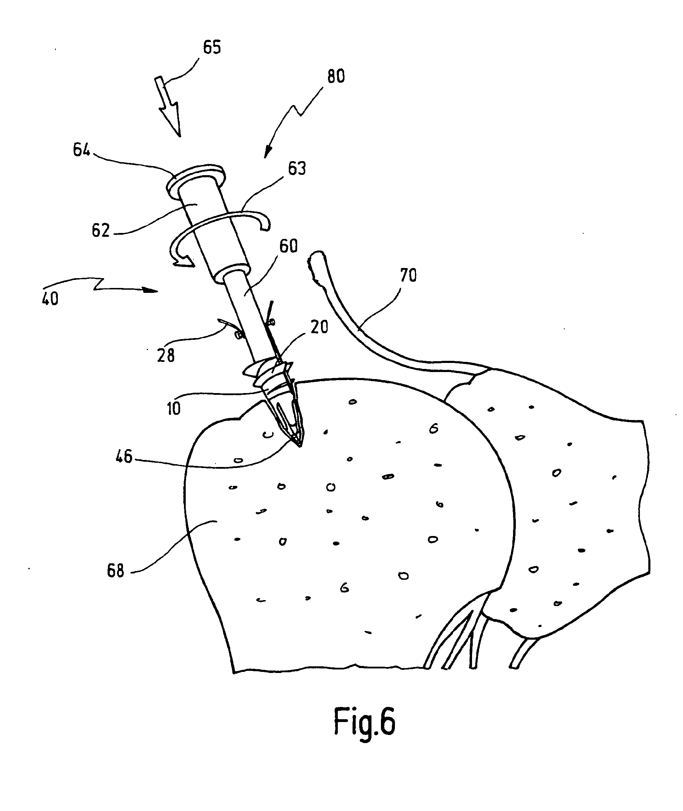

Wie aus Fig. 6 zu entnehmen ist, geht der stabförmige Abschnitt 60 in einen Griff 62 über, der am Ende mit einem Schlagkopf 64 versehen ist.

Zum Einbringen des Ankerelements 10 wird zunächst, wie in Fig. 2 dargestellt, der Faden in das Ankerelement 10 eingefädelt.

Anschließend wird, wie aus Fig. 4 ersichtlich, das Werkzeug 40 eingeschoben. Durch den seitlichen Versatz der Spitze 42 schiebt diese sich am quer durch das Ankerelement 10 verlaufenden Fadenabschnitt vorbei und der Faden 28 wird zwangsläufig beim weiteren Vorschieben in den Fangschlitz 50 eingeführt.

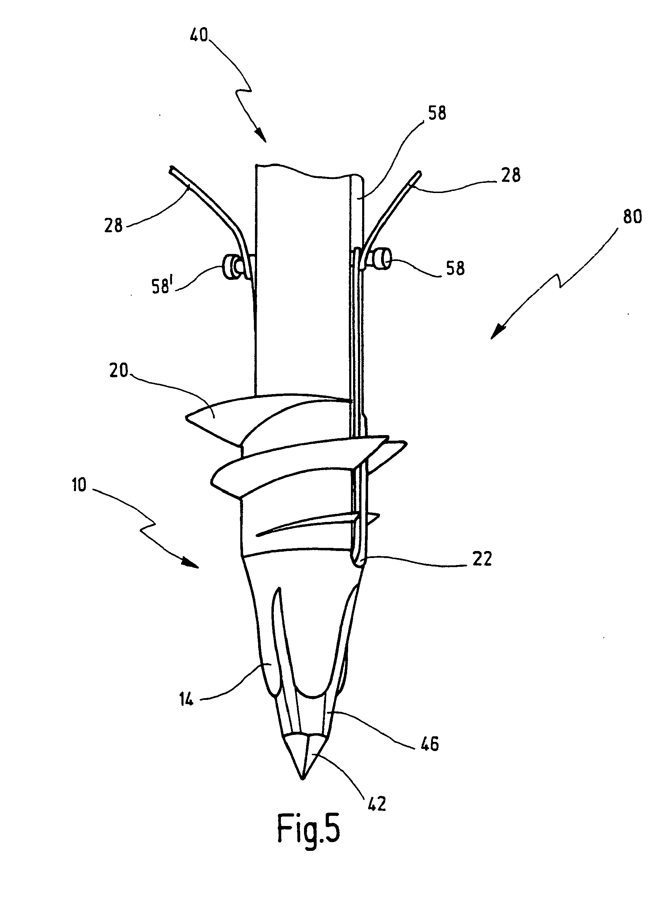

Ist das Werkzeug 40 vollständig in das Ankerelement eingeschoben, wie das aus Fig. 5 ersichtlich ist, kann der Faden 58 noch einmal straff und exakt in die Nuten 24 und 25 bzw. deren Verlängerungen 56 am Werkzeug eingelegt und über die Zapfen 58, 58' fixiert werden.

Ein distaler Endabschnitt 46 des Werkzeuges 40 ragt über das Ankerelement 10 distal hinaus. Die Verjüngung 40 bzw. die drei Segmente 16, 17, 18 sind an die Kontur dieses distalen Endabschnittes 46 angeschmiegt.

Das Werkzeug 40 ist aus einem metallischen Material hergestellt, das Ankerelement 10 aus einem resorbierbaren Material.

Zum Einbringen des Ankerelements 10 wird nun die erfindungsgemäße Vorrichtung 80, wie in Fig. 6 dargestellt, zunächst mit der Spitze 42 des vorstehenden distalen Endabschnittes 46 an einer bestimmten Stelle am Knochen beispielsweise einem Schulterknochen 68 angesetzt und die Vorrichtung 80, also der Zusammenbau aus Ankerelement 10 und Werkzeug 40, wird mit einem Schlagwerkzeug zunächst linear eingetrieben, wie das durch einen Pfeil 65 angedeutet ist.

Dieser Eintreibvorgang wird soweit durchgeführt, bis das Ankerelement 10 soweit eingetrieben ist, dass der distale Endabschnitt 46 und die Verjüngung 14 eingetrieben sind.

Nunmehr wird durch Drehen des Zusammenbaus, wie das in Fig. 6 durch einen Pfeil 63 dargestellt ist, der Zusammenbau in den Knochen 68 eingedreht, wobei dies durch das Gewinde 20 unterstützt wird. Der Faden 28 ist während dieses Vorgangs gegenüber Verschieben oder Verdrillen gesichert, da er ja in den Nuten 24, 25 bzw. 56 aufgenommen ist.

Nach dem vollständigen Eindrehen des Ankerelements 10 in den Schulterknochen 68 wird das Werkzeug 40 nach proximal abgezogen, wobei das Ankerelement 10 im Knochen verankert verbleibt.

Mit den nunmehr vorstehenden Enden des Fadens 28 kann die Sehne 70, die vom Schulterknochen 68 abgerissen ist, wieder fixiert werden.

Dadurch, dass kein 68 Bohrkanal angefertigt werden musste, liegt das Ankerelement 10 eng anliegend in dem Knochenmaterial an. Dadurch, dass es aus resorbierbarem Material hergestellt ist, wird es nach und nach durch Knochenmaterial ersetzt, so dass dann die Sehne 70 naturgetreu wieder fixiert ist.

## Patentansprüche

1. Vorrichtung zum Einbringen eines Ankerelements (10) samt Faden (28) in einen Knochen (68) zum Fixieren einer Senne (70) oder eines Bandes, ohne Bewerkstelligen einer Bohrung im Knochen (68), mit einem Ankerelement (10) und einem Werkzeug (40) zum Einbringen des Ankerelements (10) samt Faden (28), wobei ein Abschnitt (48) des Werkzeugs (40) so ausgebildet ist, dass er von proximal nach distal in das Ankerelement (10) einführbar ist, und wobei das Ankerelement (10) ein Außengewinde (20) und einen durchgehenden Kanal (30) aufweist, in den das Werkzeug (40) soweit einführbar ist, dass ein distaler Endabschnitt (46) des Werkzeuges (40) in distaler Richtung über das Ankerelement (10) hinausragt, und wobei der hinausragende distale Endabschnitt (46) als stabiler Eintreiber ausgebildet ist und an seinem distalen Endabschnitt (46) eine Spitze (42) aufweist, und wobei das Ankerelement (10) eine durchgehende Öffnung (22) zur Aufnahme des Fadens (28) aufweist, die quer zur Längsachse (36) des Ankerelements (10) verläuft, **dadurch gekennzeichnet, dass** die Lage des Endpunktes der Spitze (42) bezogen auf die Mittellängsachse (44) des Werkzeugs (40) radial versetzt ist.

2. Vorrichtung nach Anspruch 1, **dadurch gekennzeichnet, dass** das Werkzeug (40) drehschlüssig im Ankerelement (10) aufgenommen ist.

3. Vorrichtung nach Anspruch 1 oder 2, **dadurch gekennzeichnet, dass** das Ankerelement (10) distal mit einer Verjüngung (14) versehen ist.

4. Vorrichtung nach Anspruch 3, **dadurch gekennzeichnet, dass** die Verjüngung (14) so ausgebildet ist, dass diese durch die Spitze (42) des Werkzeuges (40) fortgesetzt ist.

5. Vorrichtung nach einem der Ansprüche 1 bis 4, **dadurch gekennzeichnet, dass** sich das Außengewinde (20) des Ankerelements (10) über etwa eine hintere proximale Hälfte des Ankerelements (10) erstreckt.

6. Vorrichtung nach einem der Ansprüche 1 bis 4, **dadurch gekennzeichnet, dass** sich das Außengewinde des Ankerelements über die gesamte Länge des Ankerelements erstreckt.

7. Vorrichtung nach einem der Ansprüche 2 bis 6, **dadurch gekennzeichnet, dass** der Abschnitt (48) des Werkzeuges (40) ein Querschnittsprofil aufweist, das in etwa dreisternförmig ist.

8. Vorrichtung nach einem der Ansprüche 2 bis 6, **dadurch gekennzeichnet, dass** der Abschnitt des Werkzeugs ein Querschnittprofil aufweist, das in etwa achtförmig ist.

9. Vorrichtung nach Anspruch 8, **dadurch gekennzeichnet, dass** die durchgehende Öffnung (22) in Nuten (24, 25) übergeht, die sich in axialer Richtung an der Außenseite des Ankerelementes (10) nach proximal erstrecken und die zur Aufnahme des Fadens (28) dienen.

10. Vorrichtung nach Anspruch 9, **dadurch gekennzeichnet, dass** am Werkzeug (40) Nuten (56) vorgesehen sind, die die Nuten (24, 25) des Ankerelements (10) nach proximal fortsetzen und der axialen Aufnahme des Fadens (28) dienen.

11. Vorrichtung nach einem der Ansprüche 1 bis 10, **dadurch gekennzeichnet, dass** das werkzeug (40) im Abschnitt (48) einen Fangschlitz (50) zur Aufnahme des Fadens (28) aufweist.

12. Vorrichtung nach Anspruch 11, **dadurch gekennzeichnet, dass** der Fangschlitz (50) zur einen Seite des Werkzeuges (40) mündet.

13. Vorrichtung nach Anspruch 11 oder 12, **dadurch gekennzeichnet, dass** das Ende (52) des Fangschlitzes (50) des Werkzeuges (40) auf Höhe der durchgehenden Öffnung (22) des Ankerelements (10) zum Liegen kommt, wenn das Werkzeug (40) in das Ankerelement (10) eingeführt ist.

14. Vorrichtung nach einem der Ansprüche 1 bis 13, **dadurch gekennzeichnet, dass** das Werkzeug eine Schulter (57) aufweist, die an einer entsprechenden Schulter (34) am Ankerelement (12) zum Liegen kommt.

## Claims

1. Device for inserting an anchoring element (10) and a suture thread (28) into a bone (68) to attach a tendon (70) or a ligament without drilling a hole into said bone (68), comprising an anchoring element (10) and a tool (40) for inserting the anchoring element (10) together with the suture thread (28), wherein a section (48) of the tool (40) is designed in that it can be introduced into said anchoring element (10) from proximal to distal, and wherein the anchoring element (10) has an external thread (20) and a continuous channel (30) into which the tool (40) can be inserted in such a way that a distal end section (46) of the tool (40) projects distally beyond the anchoring element (10), and wherein the projecting distal end section (46) is designed as a stable driver having a tip (42) at its distal end section (46), and wherein the anchoring element (10) has a continuous opening (22) to accommodate the suture thread (28), said opening runs transverse to the longitudinal axis (36) of the anchoring element (10), **characterized in that** the position of the end point of the tip (22) is radially offset relative to the central longitudinal axis (44) of the tool (40).

2. Device of claim 1, **characterized in that** the tool (40) is accommodated in the anchoring element (10) in a rotationally locked manner.

3. Device of claims 1 or 2, **characterized in that** the anchoring element (10) comprises a distal taper (14).

4. Device of claim 3, **characterized in that** the taper (14) is designed in such a way that it is continued by the tip (42) of the tool (40).

5. Device of anyone of claims 1 through 4, **characterized in that** the external thread (20) of the anchoring element (10) extends over roughly a rear, proximal half of the anchoring element (10).

6. Device of anyone of claims 1 through 4, **characterized in that** the external thread of the anchoring element extends over the entire length of the anchoring element.

7. Device of anyone of claims 2 through 6, **characterized in that** the section (48) of the tool (40) has a cross-sectional profile having roughly the shape of a three-pointed star.

8. Device of anyone of claims 2 through 6, **characterized in that** the section of the tool has a cross-sectional profile having roughly the shape of a figure eight.

9. Device of claim 8, **characterized in that** the continuous opening (22) continues into grooves (24, 25) which run axially, in a proximal direction, along an outside of the anchoring element (10) and which serve to accommodate the suture thread (28).

10. Device of claim 9, **characterized in that** grooves (56) are provided at the tool (40), which continues the grooves (24, 25) of the anchoring element (10) in a proximal direction, and serve to accommodate the suture thread (28) axially.

11. Device of anyone of claims 1 through 10, **characterized in that** the tool (40) has a catching slit (50) in the section (48) to accommodate the suture thread (28).

12. Device of claim 11, **characterized in that** the catching slit (50) opens to one side of the tool (40).

13. Device of claims 11 or 12, **characterized in that** the end (52) of the catching slit (50) of the tool (40) comes to rest at level of the continuous opening (22) of the anchoring element (10), when the tool (40) has been introduced into the anchoring element (10).

14. Device of anyone of claims 1 through 13, **characterized in that** the tool comprises a shoulder (57), said shoulder (57) comes to rest on a respective shoulder (34) of said anchoring element (10).

## Revendications

1. Dispositif d'introduction d'un élément d'ancrage (10) dans un os (68), conjointement à un fil (28), en vue de l'assujettissement d'un tendon (70) ou d'un ligament sans devoir pratiquer un perçage dans ledit os (68), comprenant un élément d'ancrage (10) et un outil (40) conçu pour introduire ledit élément d'ancrage (10) conjointement au fil (28), une région (48) de l'outil (40) étant réalisée de manière à pouvoir être insérée dans l'élément d'ancrage (10), de la zone proximale à la zone distale, ledit élément d'ancrage (10) comportant un filetage extérieur (20) et un canal ininterrompu (30) dans lequel l'outil (40) peut être inséré d'une distance telle qu'une région extrême distale (46) dudit outil (40) fasse saillie au-delà de l'élément d'ancrage (10) dans la direction distale, la région extrême distale saillante (46) étant conçue comme une pièce d'enfoncement stable et présentant une pointe (42) sur sa région extrême distale (46), et l'élément d'ancrage (10) étant muni, en vue de recevoir le fil (28), d'un orifice traversant (22) qui s'étend transversalement par rapport à l'axe longitudinal (36) dudit élément d'ancrage (10), **caractérisé par le fait que** la position du point extrême de la pointe (42) est décalée radialement vis-à-vis de l'axe médian longitudinal (44) de l'outil (40).

2. Dispositif selon la revendication 1, **caractérisé par le fait que** l'outil (40) est logé dans l'élément d'ancrage (10) avec assemblage par rotation.

3. Dispositif selon la revendication 1 ou 2, **caractérisé par le fait que** l'élément d'ancrage (10) est pourvu d'un rétrécissement (14) dans le sens distal.

4. Dispositif selon la revendication 3, **caractérisé par le fait que** le rétrécissement (14) est réalisé de telle sorte qu'il soit prolongé par la pointe (42) de l'outil (40).

5. Dispositif selon l'une des revendications 1 à 4, **caractérisé par le fait que** le filetage extérieur (20) de l'élément d'ancrage (10) s'étend approximativement sur une moitié proximale postérieure dudit élément d'ancrage (10).

6. Dispositif selon l'une des revendications 1 à 4, **caractérisé par le fait que** le filetage extérieur de l'élément d'ancrage s'étend sur toute la longueur dudit élément d'ancrage.

7. Dispositif selon l'une des revendications 2 à 6, **caractérisé par le fait que** la région (48) de l'outil (40) offre un profil de section transversale sensiblement configuré en étoile triple.

8. Dispositif selon l'une des revendications 2 à 6, **caractérisé par le fait que** la région de l'outil offre un profil de section transversale revêtant sensiblement la forme d'un "huit".

9. Dispositif selon la revendication 8, **caractérisé par le fait que** l'orifice traversant (22) fusionne dans des rainures (24, 25) à étendue proximale dans la direction axiale, sur la face extérieure de l'élément d'ancrage (10), qui servent à recevoir le fil (28).

10. Dispositif selon la revendication 9, **caractérisé par** la présence, sur l'outil (40), de rainures (56) qui prolongent les rainures (24, 25) de l'élément d'ancrage (10) dans le sens proximal, et servent à recevoir axialement le fil (28).

11. Dispositif selon l'une des revendications 1 à 10, **caractérisé par le fait que** l'outil (40) présente, dans la région (48), une fente d'interception (50) conçue pour recevoir le fil (28).

12. Dispositif selon la revendication 11, **caractérisé par le fait que** la fente d'interception (50) débouche vers l'un des côtés de l'outil (40).

13. Dispositif selon la revendication 11 ou 12, **caractérisé par le fait que** l'extrémité (52) de la fente d'interception (50) de l'outil (40) vient se placer à la hauteur de l'orifice traversant (22) de l'élément d'ancrage (10) lorsque ledit outil (40) est inséré dans ledit élément d'ancrage (10).

14. Dispositif selon l'une des revendications 1 à 13, **caractérisé par le fait que** l'outil comporte un épaulement (57) venant s'appliquer contre un épaulement correspondant (34), sur l'élément d'ancrage (10).
